# EUROPEAN PATENT APPLICATION

(11) **EP 4 339 228 A1**
(43) Date of publication of application: **20.03.2024**
(21) Application number: 22195433.2
(22) Date of filing: 13.09.2022
(51) Int. Cl.: C08J 3/075, C12N 1/20, C08L 5/00, C12P 19/04, C12R 1/225, C08B 37/00, C12N 9/10

(54) **STORAGE STABLE MICROBIAL COMPOSITION**

(71) Applicant: Lanbiotic GmbH, 8010 Graz (AT)
(72) Inventor: Wallner, Katrin Susanna, 8010 Graz (AT); Nidetzky, Bernd, 8010 Graz (AT); Zhong, Chao, 8010 Graz (AT)
(74) Representative: Schwarz & Partner Patentanwälte GmbH

(57) **Abstract**

The present invention relates to a method for producing a stable hydrogel-cell composition comprising the steps of:
a) providing a reaction mixture comprising at least one polymerizable substrate and at least one enzyme capable of polymerizing said at least one substrate, and cells, and
b) incubating the mixture of step a) to form a stable hydrogel-cell composition.

## Description

### FIELD OF THE INVENTION

The present invention relates to the field of hydrogel-cell compositions.

### BACKGROUND OF THE INVENTION

Three-dimensional (3D) systems comprising cell cultures have found widespread applications in the biomedical field, such as cell spheroids for studying tumour behaviours and cell encapsulation for cell-based transplantation therapies. Promising materials for cell encapsulation encompass hydrogels, soft and wet biomaterials with tunable properties, as they mimic a native extracellular matrix (i.e. an aqueous environment) having excellent mass transportation properties and showing tissue-like elasticity. These properties allow cell growth and protect the cells against environmental hazards allowing to retain cytoactivity over an extended period of time.

Various categories of hydrogels for cell encapsulation have been reported in the art. Known hydrogels typically comprise animal- or plant-derived proteins like gelatin or gluten. However, all these materials show relatively low mechanical flexibility and are thus not usable for processing. Known hydrogels may also comprise substances which can be toxic to certain types of cells. Hence, such hydrogels cannot be used for cell-based applications.

Hydrogels comprising cellulosic materials (e.g., cellulose nanofiber, cellulose nanocrystals, carboxymethyl cellulose) have been developed for cell encapsulation. Known methods for producing such hydrogels generally involve initial cellulose dissolution to form cellulose microgels, which subsequently are used for cell encapsulation. However, such methods are complicated and highly inefficient. Furthermore, in order to obtain the desired physico-chemical properties the cellulosic material has to be modified or combined with other materials. Chemical methods for cross-linking cellulose involve toxic chemical cross-linkers and catalysts such as 2,2,6,6-tertramethylpiperdine-1-oxyl radicals. These toxic chemicals negatively influence the viability of cells encapsulated by such cellulosic materials.

The drawbacks of hydrogels known in the art prevented the development of stable hydrogel-cell compositions so far. Hence, it is an object of the present invention to provide means and methods to overcome the drawbacks of the prior art for producing a stable and biocompatible hydrogel-cell composition which can be used, for instance, for tissue engineering and biomedical applications such as bioprinting.

### SUMMARY OF THE INVENTION

Thus, the present invention relates to a method for producing a stable hydrogel-cell composition comprising the steps of:
**a)** providing a reaction mixture comprising at least one polymerizable substrate and at least one enzyme capable of polymerizing said at least one substrate, and cells, and
**b)** incubating the mixture of step a) to form a stable hydrogel-cell composition.

Surprisingly, it turned out that cells can be stabilized in a hydrogel if the hydrogel is enzymatically synthesized in the presence of the cells. The inventive bottom-up strategy to synthesize the hydrogel allows to stabilize the cells embedded in the hydrogel for a much longer period of time compared to methods which include steps where a hydrogel is simply contacted with cells.

The method of the present invention allows further to form a mechanically stable hydrogel showing an excellent biocompatibility during the whole process. Due to the enzyme reaction leading to in situ gelation, cells can be embedded/encapsulated within the hydrogel to be formed more homogeneously and more efficiently. As mentioned before, this embedding/encapsulation method prolongs significantly the viability of the cells comprised within the hydrogel.

The use of polymerizable substrates and enzymes to form the hydrogel is also advantageous because it allows the creation of a biocompatible hydrogel matrix which is beneficial for the cells embedded/encapsulated in the hydrogel and the potential medical use of the inventive hydrogel-cell composition.

Thus, the inventive method for preparing hydrogels is perfectly applicable for cell embedding/encapsulation in view of a) obtaining homogeneous distribution of cells within the hydrogel due to in-situ gelation and encapsulation by premixing of cells in aqueous reaction solution, b) the mild conditions used for gel synthesis which retain cell viability during encapsulation, and c) the biocompatible and ecological friendly materials used in the hydrogel composition that make the system applicable to human- and animal-based applications. Furthermore the inventive method is a fast, simple and cost-effective process that does not require specialised equipment and makes the technology accessible to people who do not have expertise or laboratory equipment (e.g. in a pre-formulated kit) .

Hydrogels obtainable with the method of the present invention can also be used in tissue engineering. There, the hydrogels take over mechanical tasks as well as other tasks of an extracellular matrix. Water insoluble parts can be dissolved enzymatically when the growing cells excrete their own extracellular matrix. As the degradation process must be controllable, cellulose fibers, for instance, are especially suitable for cells, preferably eukaryotic cells lacking own cellulase.

Another aspect of the present invention relates to a method for producing a stable hydrogel composition comprising the steps of:
a) providing a reaction mixture comprising at least one polymerizable substrate and at least one enzyme capable of polymerizing said at least one substrate, and xanthan, and
b) incubating the mixture of step a) to form a stable hydrogel composition.

It turned out, that the addition of xanthan to the reaction mixture used in the method of the present invention leads to the synthesis of a stable hydrogel having superior mechanical properties compared to hydrogel compositions comprising other added polymers based on e.g., polysaccharides. Furthermore, it has turned out that hydrogels produced by the method of the present invention retain their mechanical properties over a much longer period of time compared to other hydrogels, in particular to hydrogels comprising other polymers (e.g., other polysaccharides). The addition of xanthan to a hydrogel produced by the method of the present invention gives further mechanical stability to said hydrogel. This enhanced mechanical stability of the hydrogel combination significantly increases the areas of use of this combination. A further advantage of the hydrogel comprising xanthan is its biocompatibility which allows to stabilize biological molecules (e.g., enzymes) or cells embedded/encapsulated therein much more efficiently compared to other additives. This leads to products having an even longer shelf-life compared to hydrogels without xanthan since the biological and enzymatic activity is retained over a longer period of time within said hydrogel combination.

Another aspect of the present invention relates to a hydrogel-cell composition obtainable or obtained by the method of the present invention.

A further aspect of the present invention relates to the *Lactococcus lactis* strain 10123 deposited on February 04 2022 with the DSMZ under deposit number DSM 34156.

*L. lactis* strain 10123 shows surprising inhibiting properties against various bacterial species of the Staphylococcus genus, for instance, such as *S. aureus*, *S*. *hominis* and *E*. *hirae.*

*L. lactis* is a well-known probiotic organism that has been widely used for thousands of years for different purposes such as cheese production. *L. lactis* is also known for bacteriocin production (such as the lantibiotic nisin), which can act as an inhibitor against certain kinds of other pathogenic bacteria such as bacteria of the genus staphylococcus. However, common *Lactococcus lactis* bacteria release only a limited amount of antimicrobial active peptides that cause inhibition in direct confrontation with other germs. Hence, it is a surprising finding that in particular *L. lactis* strain 10123 exhibits excellent antibiotic properties towards many microorganisms compared to known *L. lactis* strains.

The strain of the present invention shows to be surprisingly effective also against antibiotic-resistant microorganisms. The rapid emergence of antibiotic-resistant microorganisms, in particular bacteria, is occurring worldwide, endangering the efficacy of antibiotics, which have transformed medicine and saved millions of lives. Many decades after the first patients were treated with antibiotics, bacterial infections have again become a threat. Hence, the provision of means and methods for fighting these antibiotic-resistant microorganisms is of major importance. *L. lactis* strain 10123 turned out to be able to prevent and/or inhibit the growth of antibiotic-resistant microorganisms.

Particularly *S. aureus* and its antibiotic resistant form methicillin-resistant *Staphylococcus aureus* (MRSA) is a human pathogen of concern and the cause for e.g., skin infections, respiratory infections, and food poisoning. For instance, the elimination of *S. aureus* dysbiosis, a severe skin disease, by antibiotics shows promising improvements of atopic skin condition in different studies. The use of the strain of the present invention in systemic and topical medicines could be a useful application to combat the spread of multidrug-resistant germs.

Another preferred application of the strain of the present invention refers to cosmetic goods. Up to now, cosmetic products have mainly used prebiotic (favourable skin flora) or postbiotic (lysate or bacterial metabolites) agents. Also, bacterial components or dead bacteria have immunomodulatory effects. The use of alive bacteria, however, brings additional advantages. This is particularly important in the direct inhibition of pathogens such as *C*. *albicans* or *S. aureus.*

*L. lactis* strain 10123 turned out to produce sufficient quantities of antimicrobialactive substances so that the strain can be used also as food additive (e.g., as preservative). Another aspect of the present invention relates to a composition comprising *Lactococcus lactis* 10123.

Another aspect of the present invention relates to a kit for producing a stable hydrogel-cell composition comprising at least one polymerizable substrate and at least one enzyme capable of polymerizing said at least one substrate.

### SHORT DESCRIPTION OF THE FIGURES

Fig. 1 shows a scheme of beta-1,4-glycosylation of cellobiose using alpha-D-glucose-1-phosphate (aGlc1P) as the donor catalyzed by cellodextrin phosphorylase (CdP).
Fig. 2 shows the reaction mixtures containing various xanthan concentrations (0-0.5%, m/v). Reactions were performed using 25 mM cellobiose, 200 mM Glc1-P, 0.5 mg/mL cellodextrin phosphorylase (CdP) in 10 mM phosphate buffer (pH 7.0) at 37 °C, 3 h.
Fig. 3 shows the profile of amplitude sweep analysis of synthetic cellulose-hydrogel with 0.4% xanthan.
Fig. 4 shows a confocal laser scanning microscopy photograph of living cells (white) encapsulated within hydrogel.
Fig. 5a shows the CFU of the bacteria (*Lb. rhamnosus*) in the gel in the course of storage time.
Fig. 5b shows the bacterial survival (*Lb. rhamnosus*) in standard hydrogel (xanthan only) in the course of storage time.
Fig. 6a shows the CFU of the bacteria (*L.Lactis*) in the gel in the course of storage time.
Fig. 6b shows a comparative analysis of cell survival between (*Lb. rhamnosus*) embedded in a standard hydrogel (xanthan) and *L. lactis* in hydrogel with xanthan according to the present invention.
Fig. 7 shows inhibition zones on Müller-Hinton-agar inoculated with *S. aureus.*
Fig. 8 shows the inhibition of *S. aureus* in co-culture with *L. lactis* 10123 in comparison to a *S*. *aureus*-monoculture and a *S. aureus* co-culture with *Lb. rhamnosus* after 24 hours of incubation without *L. lactis* 10123.
Fig. 9a shows a blood agar plate inoculated with 10^6 CFU *S. aureus* with 10^7 CFU/ml *L. lactis* on the top.
Fig. 9b shows an inhibition zone of a blood agar plate where the hydrogel drop with *L. lactis* had been applied directly.
Fig. 10 shows a porcine skin model inoculated with *S*. *aureus* (left spot) and with *S. aureus* and *L. lactis* (right spot).

### EMBODIMENTS OF THE INVENTION

A "stable hydrogel-cell composition", as used herein, refers to a composition comprising at least one hydrogel and (biological) cells embedded and/or encapsulated in said at least one hydrogel. "Stable" in regard to said compositions means that the physicochemical properties of the hydrogel remain substantially unchanged for a period of at least 30, preferably at least 60, more preferably at least 180 days, at a temperature between 2°C and 30°C, preferably between 4°C and 25°C, even in the presence of the cells embedded and/or encapsulated in the hydrogel. Furthermore, "stable" refers also to the viability of the cells encapsulated and/or embedded in the hydrogel and retaining their functions. With the method of the present invention, it is possible to produce hydrogel-cell compositions where the cells embedded and/or encapsulated therein are viable for at least 30, preferably at least 60, more preferably at least 180 days, at a temperature between 2°C and 30°C, preferably between 4°C and 25°C. The number of viable cells embedded and/or encapsulated in the hydrogel obtainable by the method of the present invention decreases minimally under these conditions. It could be shown that the number of viable cells (CFU, colony forming units) decreased even over a period of 6 months minimally (less than 2 log CFU/ml) at room temperature. The stress tolerance of the composition was also tested by performing 3 freeze-thaw cycles where the composition was stored at -18 degree for 24 hours, following storage at room temperature for the next 24 hours, following storage at 37 degree for 24 hours and finally storing at room temperature for 24 hours. The viability of the cells can be determined by enumerating the encapsulated cells on agar plates according to Arepally D. et al (Curr Res Food Sci. 2020 Oct 1;3:235-242). However, other methods are known in the art to determine the number of CFUs. Depending on the type of cells, different methods may be used.

A "polymerizable substrate", as used herein, refers to at least one molecule capable of forming a polymeric network upon mixture with at least one polymerizing enzyme.

The reaction mixture comprising at least one polymerizable substrate and at least one enzyme capable of polymerizing said at least one substrate, and cells is incubated at conditions allowing the enzymatic polymerisation of its substrates and at conditions which do not negatively affect the viability or function of the cells to be embedded and/or encapsulated. Hence, the conditions depend on the enzyme and on the cells employed in the method of the present invention. These conditions are well-known to a person skilled in the art.

Another aspect of the present invention relates to a method for producing a stable hydrogel composition by providing and incubating a reaction mixture comprising at least one polymerizable substrate, at least one enzyme and xanthan.

Xanthan is known a common food additive and plays an important role in industrial applications as thickener, emulsion stabilizer and is usually added to water-based drilling fluids due to its pseudoplastic behaviour and thermal stability. The present invention shows that the addition of xanthan to the reaction mixtures significantly contributes to enhanced cell biocompatibility which is more environmentally friendly and safe, even for human consumption, compared to other chemical additives such as PEG. The formed stable hydrogel composition comprising a reaction mixture and xanthan could therefore be used as a suitable carrier for cell encapsulation. The addition of xanthan results surprisingly also in a much better storage-stability of hydrogel-cell compositions, since the viability of the cells within such compositions can be better preserved in the presence of xanthan.

According to a preferred embodiment of the present invention, the reaction mixture for producing a stable hydrogel composition further comprises cells.

According to a preferred embodiment of the present invention, the at least one polymerizable substrate is a sugar phosphate, preferably a hexose phosphate, more preferably a glucose phosphate, even more preferably alpha-D-glucose-1-phosphate.

The use of sugar as polymerizable substrates in the methods of the present invention is particularly preferred. Sugars are known components of carbohydrate-based hydrogels. Sugar monomers can be linked to each other by using respective enzymes capable of polymerizing said sugar monomers. The bottom-up synthesis from monomers to oligomers and polymers is turned out to be advantageous because it allows to embed cells within the polymer matrix. Since most of the enzymes capable of polymerizing sugar monomers require phosphorylated sugars, it is particularly preferred to use sugar phosphates as monomers.

In case the at least one polymerizable substrate is a sugar phosphate like alpha-D-glucose-1-phosphate, sucrose can be added to the reaction mixture of step a) or can be used in a separate reaction step to form alpha-D-glucose-1-phosphate, as described by Zhong and Nidetzky (Biotechnol. J. 2020, 15, 1900349).For instance, sucrose phosphorylase catalyzes the formation of alpha-D-glucose-1-phosphate and fructose from sucrose and phosphate ions.

According to another preferred embodiment of the present invention, the alpha-D-glucose-1-phosphate is formed by incubating sucrose with sucrose phosphorylase. The use of sucrose phosphorylase for producing alpha-D-glucose-1-phosphate is particularly advantageous because sucrose is much more economic compared to, e.g., alpha-D-glucose-1-phosphate. The use of sucrose as polymerizable substrate avoids the use of alpha-D-glucose-1-phosphate, making the production of the stable hydrogel composition much more cost-efficient.

The stable hydrogel composition of the present invention can also be produced in a one-pot reaction together with sucrose phosphorylase, phosphate ions and sucrose as substrate. Thus, the reaction mixture of the method of the present invention may comprise next to the at least one polymerizable substrate and the at least one enzyme capable of polymerizing said at least one substrate sucrose phosphorylase, phosphate ions and sucrose. It turned out that the components of such a reaction mixture do not affect negatively each other.

"A one-pot reaction", as used herein, refers to a strategy to improve the efficiency of a chemical reaction whereby the reactants are subjected to just one reaction mixture. This is much desired because a one-pot reaction may avoid a lengthy separation process and purification of the intermediate compounds can save time and resources while increasing production yield.

According to another preferred embodiment the reaction mixture comprises at least one further monosaccharide, oligosaccharide and/or polysaccharide, preferably at least one further oligosaccharide and/or polysaccharide.

The polymerizable substrate to be used in the method of the present invention is preferably a sugar monomer. However, in order to increase the polymerisation rate it is possible to add at least one further monosaccharide, oligosaccharide and/or polysaccharide to which a sugar monomer can be attached by the enzyme used.

According to another further preferred embodiment the at least one further monosaccharide is glucose and/or the at least one further oligosaccharide is a disaccharide, preferably cellobiose. Cellobiose, for instance, can be obtained by enzymatic or acidic hydrolysis of cellulose and cellulose-rich materials such as cotton, jute, or paper. It turned out that building up a cellobiose chain via precision polymerization offers exceptional tunability and control of the final chemical structure.

Depending on the reaction conditions leading to different degrees of polymerization (DP), long-chain soluble oligosaccharides or insoluble materials can be formed.

According to a preferred embodiment of the present invention, the reaction mixture comprises 10 to 500 mM, preferably 15 to 400 mM, more preferably 20 to 300 mM, of said at least one polymerizable substrate and/or of said at least one further monosaccharide, oligosaccharide or polysaccharide.

According to a further preferred embodiment of the present invention a mixture of α-D-glucose 1-phosphate (alpha-D-glucose 1-phosphate) and cellobiose is used as a polymerizable substrate. Cellobiose can be glycosylated via beta-1,4-glycosylation using alpha-D-glucose 1-phosphate as the donor substrate. The alpha-D-glucose 1-phosphate donor substrate can be made available efficiently through an additional phosphorolysis reaction by enzymes that use starch or sucrose as the substrate.

It turned out that the degree of polymerization may depend on the molar ratio of the cellobiose acceptor and alpha D-glucose 1-phosphate donor used in the reaction. An efficient synthesis of a hydrogel requires good control over the DP of the products formed. Hence, the reaction mixture may comprise alpha-D-glucose-1-phosphate and cellobiose as polymerizable substrate, preferably in a molar ratio from 20:1 to 1:1, preferably from 10:1 to 2:1, more preferably from 8:1 to 5:1. In particular these ratios turned out to be advantageous in forming a hydrogel which is able to stabilize cells embedded therein.

To initiate the polymerization of the reaction mixture, at least one phosphorylase can be used. Phosphorylases are enzymes that catalyze the addition of a phosphate group from an inorganic phosphate donor to an acceptor. Phosphorylase reactions typically involve scant hydrolysis in consequence of exclusion of reaction with water from the enzyme active site. Consequently, phosphorylases may contribute to the synthetic building up of oligosaccharide or polysaccharide chains which then undergo oligomerization-induced self-assembly into a hierarchically organized and property-tunable materials.

Thus the at least one enzyme capable of polymerizing the aforementioned substrates is preferably a phosphorylase, more preferably an oligosaccharide phosphorylase or a polysaccharide phosphorylase.

In particular, the enzymatic reaction of the method of the present invention may be catalysed by the phosphorolysis of beta 1,4 glycosidic bonds oligosaccharide substrates resulting in polysaccharide chains built from several hundred or more linked glucosyl groups. Phosphorylase can act on the beta 1,4 glycosidic linkage in oligo- and polysaccharide substrates.

According to a preferred embodiment of the present invention, said at least one enzyme is cellodextrin phosphorylase.

For instance, cellodextrin phosphorylase (EC 2.4.1.49) can reversibly catalyze synthesis of cellulose and free phosphate (products) by iterative beta-1,4-glycosylation from alpha-D-glucose-1-phosphate and short-chain cellodextrin (as substrates). It is particularly preferred to use bacterial cellodextrin phosphorylase, preferably from Clostridia, more preferably from *Clostridium cellulosi* (e.g., GenBank ID CDZ24361.1) .

According to another preferred embodiment of the present invention the reaction mixture comprises 0.5 to 50 U/ml, preferably 0.5 to 30 U/ml, more preferably 1 to 20 U/ml, more preferably 1 to 10 U/ml, of said at least one enzyme.

According to another preferred embodiment of the present invention the reaction mixture comprises at least one further carbohydrate polymer, preferably in an amount of 0.1 to 1 wt% of the reaction mixture. Carbohydrate polymers are composed of long chains of carbohydrate monomers attached via glycosidic bonds. Carbohydrate polymers can be added to said reaction mixture of the present invention. Depending on the added carbohydrate polymer, the properties of the obtained polymer composition can be adjusted without the use of UV light or temperature changes.

According to another preferred embodiment of the present invention the at least one further carbohydrate polymer comprises carboxylic groups, acetyl groups and/or sulphate groups.

Modified carbohydrate polymer can be added to the reaction mixture in order to introduce further properties to the hydrogels. Modifications of carboxylic groups, for instance, may lead to changes in hydrolysis resistance as well as the thermal stability of polymers. Sulphate groups bound to sugar residues may contribute to the stabilization of polymer compositions by introducing terminal sulphate groups on each polymer chain formed.

According to another preferred embodiment of the present invention at least one further carbohydrate polymer is xanthan.

Xanthan is a heteropolysaccharide primarily composed of D-glucose and D-mannose as the dominant hexose units, along with D-glucuronic acid and pyruvic acid. Xanthans can chemically increase the viscosity of the liquid it is mixed into. Because of this ability, e.g., xanthan gums are frequently used in the food industry as an ingredient in sauces and salad dressings as well as in cosmetics. It turned out surprisingly that the addition of xanthan to the reaction mixture of the present invention enhances the mechanical stability of the hydrogel and prolongs the viability of the embedded cells. In addition, xanthan is an environmentally friendly ingredient and a surprising substitute to other chemical ingredients and stabilizers that are used in in-situ hydrogel gelation such as PEG.

The formed stable hydrogel composition is built up from the polymerizable substrate as a basis that interacts and/or is associated with xanthan.

According to another preferred embodiment of the present invention the reaction mixture is obtained by combining its components in buffer solution, preferably in a phosphate-buffered saline (PBS) buffer solution, preferably having a pH of 6.5 to 7.5. An isotonic buffer solution having a neutral pH can be used as an optimal composition to maintain the enzymatic activity of e.g., phosphorylase and to be non-toxic to most cells.

One surprising effect achievable with the method of the present invention is the superior viability of embedded prokaryotic and eukaryotic cells in the hydrogel composition. In addition, overgrowth of the cells in the hydrogel may be limited and viability remains stable over time. According to a preferred embodiment of the present invention, the cells are bacterial cells, fungal cells, animal cells or human cells.

According to another preferred embodiment of the present invention the bacterial cells are of the order of *Lactobacillales*, preferably of the family of *Lactobacillaceae* and/or of the family of *Streptococcaceae*, more preferably of the genus *Lactococcus* and/or of the genus *Lactobacillus.*

According to another further preferred embodiment of the present invention the bacterial cells are selected from the group consisting of *Lactococcus lactis*, preferably *Lactococcus lactis* 10123 (DSM 34156), *Lactobacillus rhamnosus.*

According to another preferred embodiment of the present invention the reaction mixture comprises the cells in a concentration of 0.5 to 50 mg/ml, preferably 0.5 to 40 mg/ml, more preferably 0.5 to 30 mg/ml, more preferably 1 to 20 mg/ml, more preferably 1 to 10 mg/ml. The CFU/mg can be between 1,0 * 10^3 and 1,0 * 10^12, preferably between 1,0 * 10^4 and 1,0 * 10^10. The CFU/mg may also depend on the type of cells and the purpose for which the hydrogel-cell composition is used.

According to another preferred embodiment of the present invention lyophilized cells are added to the reaction mixture.

Lyophilization, also known as freeze-drying, is a process used for preserving biological material by removing the water from the sample, which typically involves the optional addition of lyoprotectants (e.g. sugar alcohols like mannitol, sorbitol or xylitol) followed by freezing the sample and then drying it, under a vacuum, at very low temperatures. By adding lyophilized cells to the reaction mixture, cultivation time and viability of the embedded cells can be extended. Lyophilization may stabilize the embedded cell cultures for long-term storage while minimizing the damage that may be caused by strictly drying the sample.

According to another preferred embodiment of the present invention the mixture of step a) is shaken during step b) at 50 to 1000 rpm, preferably at 100 to 800 rpm, more preferably at 200 to 600 rpm, more preferably at 200 to 500 rpm. Shaking of the mixture may contribute to a homogenous distribution of ingredients of said mixture.

Another aspect of the present invention relates to a hydrogel-cell composition obtainable by a method as described above. Such compositions with encapsulated and/or embedded cells showed long term stability at room temperature with viable cells for at least 30, 60 or even 174 days, while preserving the gel properties in the presence of the cells. The obtained composition is therefore an easy solution for keeping the encapsulated and/or embedded cells alive with enhanced shelf life in comparison to other in-situ hydrogels.

According to a preferred embodiment of the present invention, the hydrogel-cell composition is contacted on a patch or wrap for topical application on the skin, mucosa or tissue inside of the body such as organ surfaces. The adhesion of the composition as part of the patch or wrap to the body is advantageous because it allows a controlled and constant administration of the hydrogel-cell composition.

The hydrogel-cell composition stays at the site of action until the patch/warp is removed and the patch also fulfils adhesive tasks such as wound closure. Administration of the composition through a patch or wrap optimizes the amount and speed at which molecules are absorbed by the body and becomes available at the site of action. Furthermore, the patch or wrap may prevent early dehydration of the hydrogel, harmful infections of e.g., wounds and enables an easy and facile use of the composition by self-administration. It does not require expert personnel for its placement, it can be applied on e.g., the skin on the area according to user's needs. The patch or wrap structure can be configured to allow to be worn directly on the skin of the user and can be adapted according to the required site of action.

According to another preferred embodiment of the present invention, the hydrogel-cell composition can be used in food production. The composition may comprise microbial cells such as bacteria or fungi which can contribute in the production of e.g., milk products such as cheese yoghurt, meat products such as salami, or food supplements. The role of microbial cells in food production are known in the art.

The hydrogel-cell composition of the present invention may be further used in preserving food products through formation of inhibitory metabolites such as organic acid (e.g. lactic acid, acetic acid, formic acid or propionic acid) or ethanol. The hydrogel-cell composition may help to improve food safety through inhibition of pathogens and removing toxic compounds. Further, the composition of the present invention can improve the nutritional value and the organoleptic quality of food.

According to another preferred embodiment of the present invention, the hydrogel-cell composition can be used in agriculture and/or farming. Further compositions which regularly are used in the cultivation of crop plants can be mixed with said hydrogel. The composition can be selected from the group consisting of pesticides, herbicides, insecticides, molluscicides, fungicides, pheromones and preferably, fertilizers.
Another aspect of the present invention relates to a hydrogel-cell composition for the use in the treatment of a disorder or an injury of the skin and/or the eye.

Due to the unique properties of the hydrogel-cell composition of the present invention resulting in a significant prolongation of the viability of the cells embedded in the hydrogel, the hydrogel-cell composition of the present invention or obtainable by a method of the present invention can be used in the treatment of disorders or diseases associated with the skin and/or eye. The hydrogel-cell composition may be contacted directly with the skin and/or eye to promote the healing and/or tissue regeneration process. The cells present in the hydrogel-cell composition are selected to have therapeutic effects on the skin and/or eye. Respective cells are well-known in the art.

Due to the mechanical and cytoprotective properties of the composition of present invention said hydrogel-cell composition can flexibly adapt to a defect's shape on the skin and/or the eye and enhance proper wound closure, for instance, and the tissue regeneration process by the presence of cells or their respective secretomes. After administration, the inventive composition aligns to a defect's form (e.g., a wound) during in-situ polymerization promoting the healing process.

According to another preferred embodiment of the present invention, the cells in the hydrogel-cell composition are selected from the group consisting of stems cells, induced pluripotent stem cells, macrophages, keratinocytes, endothelial cells, T-lymphocytes, monocytes, oozytes, spermatozoon and combinations thereof. Some of these cell types are associated with wound healing and tissue remodelling and can be used in the treatment of skin and/or the eye disorders/diseases. Also, the secretome of some of these cells may contribute significantly to the healing of wound and tissue repair mechanisms, for instance. Suitable secretomes comprise various growth factors (e.g., PDGF, TGF-β, β-FGF) and cytokines (e.g., NF-α, interleukin 1 (IL-1), and IL-6) that are known to promote healing of the skin and/or the eye.

According to another preferred embodiment of the present invention the hydrogel-cell composition can be administered topically and/or by injection onto or into the skin or eye, preferably through subcutaneous or intravitreal injection.

The present invention can be used for the treatment of a disorder or an injury of the skin and/or the eye.

The term "injury", as used herein refers to a physical trauma experienced by the skin, the eye or multiple layers of epithelial tissues. This can be in the form of cuts, shocks, burns, scratches, and/or scrapes.

According to another preferred embodiment of the present invention the disorder or injury of the skin and/or the eye is selected from the group consisting of acne, alopecia areata, atopic dermatitis (eczema), psoriasis, raynaud's phenomenon, rosacea, vitiligo, acute wounds, chronic wounds, corneal abrasion, glaucoma, cataract, burns and/or irritation of the skin/and or the eye, actinic keratosis, melanoma, sun-damaged or aging skin and combinations thereof.

Another aspect of the present invention relates to a method for the use in the treatment of a disorder or an injury of the skin and/or the eye.

According to another preferred embodiment of the present invention, a composition may comprise *Lactococcus lactis* 10123. The composition of the present invention has shown surprising inhibiting effects on pathogens, in particular on pathogens of the genus *Staphylococcus* like *S. aureus.* Further, said composition can be mixed with the reaction mixture and least one enzyme. It turned out that the strain *Lactococcus lactis 10123* is suitable to be embedded in said obtained hydrogel composition as described above.

Another aspect of the present invention relates to a composition obtainable by a method according to the present invention. The composition the present invention may be effective on pathogenic germs such as *S. aureus* and could be used as a substitute for antibiotics in the food industry, agricultural industry, medicine as well as the cosmetic area. The composition of the present invention can be used in the treatment or prevention of skin infections caused by various pathogens, in particular caused by bacterial or fungal pathogens. It turned surprisingly out that compositions obtainable by the method of the present invention and comprising *Lactococcus lactis 10123*, for instance, can be used effectively to inhibit or prevent the growth of pathogens on the skin and/or wounds. In addition, the composition of the present invention can be used for the strengthening of the skin and mucous membrane barrier function of the skin and for inducing anti-inflammatory behaviour in human cells by altering genetic expression and cytokine segregation. For instance, the relatively low pH due to presence of *Lactobacilli* not only promotes the natural protective acidic environment of the skin, but also inhibits the growth of harmful bacteria, viruses, and fungi. These properties make the composition of the present invention an ideal product for the use as a hygiene product, preferably as an intimate hygiene product. Many women experience problems especially after a bladder infection treated with a broad-spectrum antibiotic. Since this treatment regime kills many different germs, it also affects the natural intestinal and vaginal flora, resulting in a bacterial miscolonization of the mucous membranes of the vagina, rectum and perineum. These bacteria can cause alterations in the pH value in the mucous membranes and thus, weakening the natural protective function against pathogens. The composition of the present invention may also have antiviral and/or anti-tumoral effects, thus, preventing the formation of tumours caused by viruses like human papillomavirus. The composition of the present invention may prevent the formation of carcinoma of the skin, the eye and or the mucous membrane. After topical application of the composition, the occurrence of precancerous conditions can be prevented due to the positive influence of *Lactobacilli* on the tumor microbiome. In addition, the composition may also be used for the inhibition of the proliferation of viruses leading to abnormal cell growth causing precancerous lesions, cancer, or genital warts. The composition of the present invention may also contribute to balance the natural skin and mucous membrane flora by maintaining the physiological pH and inhibiting the proliferation of harmful bacteria and fungi. According to another preferred embodiment of the present invention, the composition can be in a form suitable for topical application, such as an ointment, a creme, a gel, a lotion, a powder or an emulsion. Emulsions can be prepared by a high shear mixing process from at least two phases, such as a hydrophilic (aqueous) and a lipophilic (oil) phase.
It turned surprisingly out, that the composition of the present invention in the form of an emulsion can be prepared by using ingredients that have been experimentally shown to be compatible with bacteria such as *Lactococcus lactis 10123.* Compatibility is measured by stirring *L. lactis* lyophilisate into each ingredient and measuring CFU per ml or per gram on blood agar after 7 days.

According to a preferred embodiment of the present invention, the emulsion can be prepared by
**a)** providing an oil phase and an aqueous phase by mixing and heating each phase separately,
**b)** mixing the phases, and
**c)** cooling the mixture.

The first step involves mixing and heating the oil phase, which comprises e.g., shea butter and/or sunflower seed oil. In the second step, the aqueous phase, comprising purified water, salts and gelling agents such as xanthan gum as well as an emulsifier such as lecithin, is prepared and heated. In the third step, the two phases are mixed under high shear and cooling. A stable formulation can be produced, which can be mixed carefully with the stable hydrogel composition of the present invention. The hydrogel has been shown to be mechanically stable for at least three months when mixed into the emulsion of the present invention.

According to another preferred embedment of the present invention, further active ingredients can be added to the emulsion. Active ingredients can comprise ceramides, ingredients that serve as a food source for bacteria, such as glucose, or buffers and acids such as lactic acid.The composition of the present invention can also be applied on the skin as well as orally, intraorally, vaginally or rectally or within areas of operation inside of the body. The compositions of the invention may be used for a variety of conditions, prophylactically or curatively.

According to a further preferred embodiment of the present invention, the composition in the form of an ointment, a creme, a gel, a lotion, a powder or an emulsion can be contacted on a patch or wrap for topical application on the skin. The composition can be applied as part of a patch or wrap onto the skin where delivery of microorganisms to a targeted area of the skin is intended. The use of a patch or wrap may even more prolong the survival of the bacteria during application, by preventing dehydration and/or infections.

Another aspect of the invention relates to a kit for producing a stable hydrogel-cell composition. The kit can be multicomponent system comprising at least one polymerizable substrate and at least one enzyme capable of polymerizing said at least one substrate as described herein. After mixing the components as described above, the hydrogel composition of the present invention can be obtained.

According to another preferred embodiment, the at least one polymerizable substrate is defined as described above.

According to another preferred embodiment the at least one enzyme is defined as described above.

According to another further preferred embodiment the kit further comprises cells, in particular cells as described above. The cells can be lyophilized and may be added to the other components of the kit.

### EXAMPLES

### Example 1: Enzymatic synthesis of the hydrogel

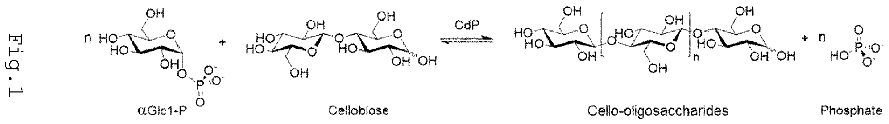

The enzyme used for hydrogel synthesis was cellodextrin phosphorylase (CdP, EC 2.4.1.49). The enzyme catalyzes the iterative β-1,4-glycosylation of cellobiose using αGlc1-P as donor substrate (Fig. 1). For this experiment, a CdP from Clostridium cellulosi (GenBank ID CDZ24361.1) was used. It was recombinantly expressed in Escherichia coli BL21(DE3) strain with a plasmid vector (pET-21b(+)) harboring codon-optimized gene. The gene was carrying an N-terminal Nde I and a C-terminal Xho I restriction site for sub-cloning into pET-21b(+). Enzyme expression in E. coli was induced by isopropyl β-D-1-thiogalactopyranoside (0.25 mM) at 18°C, overnight. Enzymes were then purified using a pre-packed (1.6 cm × 2.5 cm; 5 mL) HisTrap FF crude column (GE Healthcare Europe, Vienna, Austria) on an ÄKTA prime plus (GE Healthcare Europe). The N-terminally His-tagged proteins were eluted with imidazole (0.01 - 0.3 M). The purified proteins were desalted using phosphate buffer (50 mM, pH 7.0) with Vivaspin Turbo 50 kDa cut-off tubes (Sartorius Stedim, Vienna, Austria). The purified enzyme solution was sterilized via 0.22 µm syringe filters before using.

Synthesis reactions (0.5 mL total volume) were tested at 37°C and 45°C on a ThermoMixer C (Eppendorf, Vienna, Austria), and a temperature at 37°C was preferred. The reaction solution (RS) was prepared in the phosphate buffer solution (PBS, 10 mM, pH 7.0) containing 200 mM αGlc1-P, 25 mM cellobiose and CdP in concentration of 0.1-0.8 mg/mL (i.e., 1-10 U/mL). Reaction was processed for 1-5 hours to give stable hydrogel.

### Example 2: Enzymatic synthesis of the hydrogel with xanthan

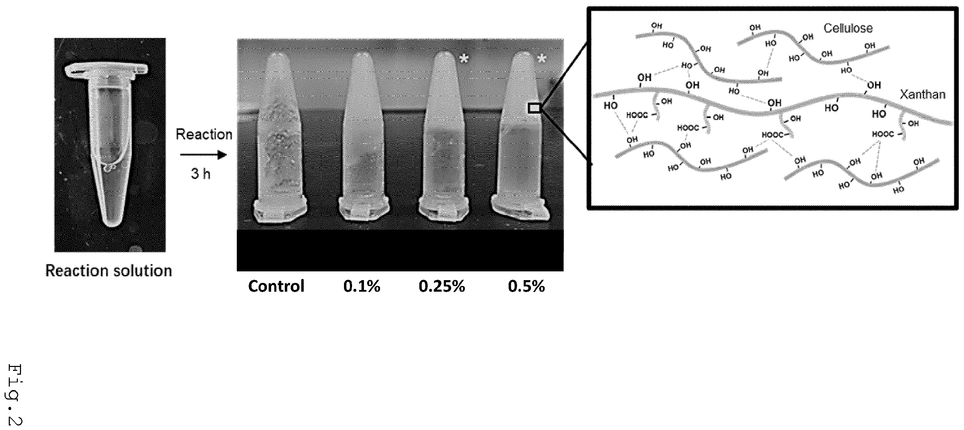

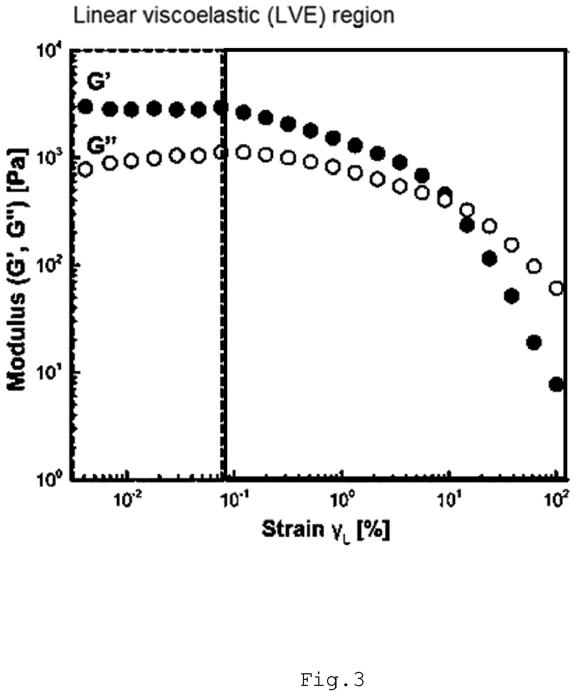

Xanthan concentrations of 0.2-0.5% (m/v) were used as additive in the aforementioned RS and a homogenous whitish hydrogel was obtained after 2 hours of reaction at 37°C (Fig. 2). Under these conditions, the conversion yield of donor αGlc1-P was around 35-55 mol.%. A control reaction carried out under identical conditions but lacking xanthan was performed for 4 h to prepare the "cellulose-only" gel, and the overall mixture showing less mechanical stability was observed (control in Fig. 2). These results thus suggested the role of polymer additives in gelation. The presence of xanthan induced a macromolecular crowding effect at a high concentration (≥ 0.25%), as shown in Fig. 2. In addition, gelation property of the thus obtained hydrogel (with 0.4% xanthan) was checked by rheological means using a strain-controlled rheometer (MCR 502, Anton Paar, Austria) at 25 °C with a cone-and-plate measurement geometry (CP 50-1) at 50 mm diameter and 1° cone. The linear viscoelastic range was measured with strain sweep (0.01-100%) at a fixed frequency of 10 rad/s. The result of amplitude sweep was presented with strain plotted on the x-axis and storage modulus G' and loss modulus G" plotted on y-axis (Fig. 3). The linear viscoelastic region (LVE region) was determined (~0.1%), wherein the curve of the G' function is a constant value that means the test is carried out without destroying the structure of the sample. The prepared hydrogel exhibited G' > G" in the LVE region, indicating that a gel-like structure was established in the hydrogel.

### Example 3: Encapsulation of cells in the hydrogel

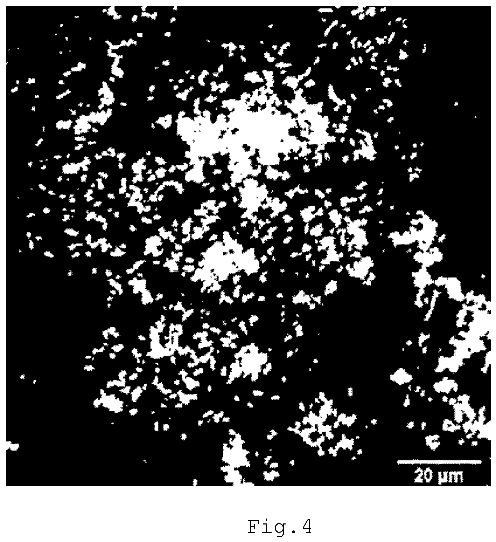

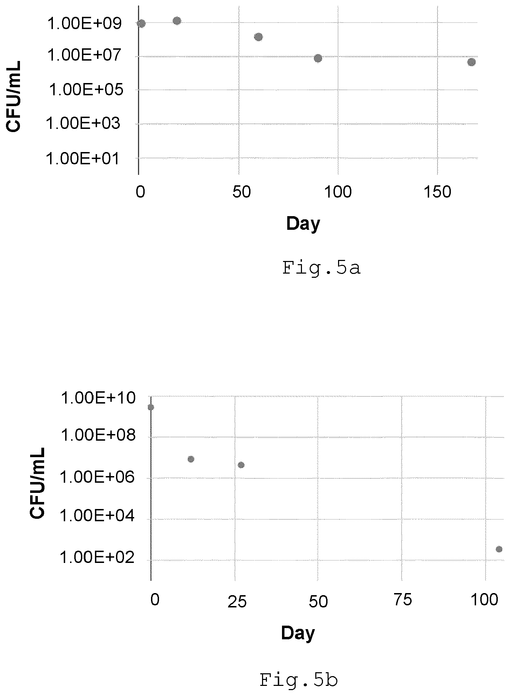

To encapsulate the cells (*Lactobacillus* and *Lactococcus* strains were tested) in hydrogel, lyophilized cells were presuspended into sterilized RS (0.5 mL) with a cell concentration in 1-10 mg/mL. Reaction was then carried out at 37°C and 300 rpm agitation on a ThermoMixer C (Eppendorf, Vienna, Austria) for 3 h. A cellulose-xanthan hydrogel with bacteria encapsulated was obtained. The overall gel property was retained in the presence of encapsulated cells. Meanwhile, a confocal laser scanning microscopy was used to visualize the encapsulated cells (*L. rhamnosus*, living cells with green fluorescence), and result showed that the cells were distributed within the hydrogel (Fig. 4). Besides, the encapsulated cells (*L. rhamnosus*) in the cellulose hydrogel without xanthan ("cellulose-only") showed long-term storage stability with viable cells of ≤ 1 log (cfu/mL) cell loss within 90-day and > 3 log (cfu/mL) cell loss after 90-day storage at room temperature. The encapsulated cells in the cellulose-xanthan hydrogel *L. rhamnosus*) exhibited long-term storage stability, with viable cells of ≤ 1 log (cfu/mL) cell loss within 60-day and ≤ 2 log (cfu/mL) loss in 90-day storage at room temperature (Fig. 5a). In comparison, the cells in xanthan solution (containing xanthan as the sole polysaccharide at a concentration of 0.1-0.5%) showed cell loss over 6 log (cfu/mL), as shown in Fig. 5b. Overall, the outstanding cytoprotection effect is achieved through encapsulation of cells in the cellulose hydrogel as well as in the cellulose-xanthan hydrogel.

### Example 4: Hydrogel encapsulation for preserving viability of cells (L. rhamnosus and L. lactis)

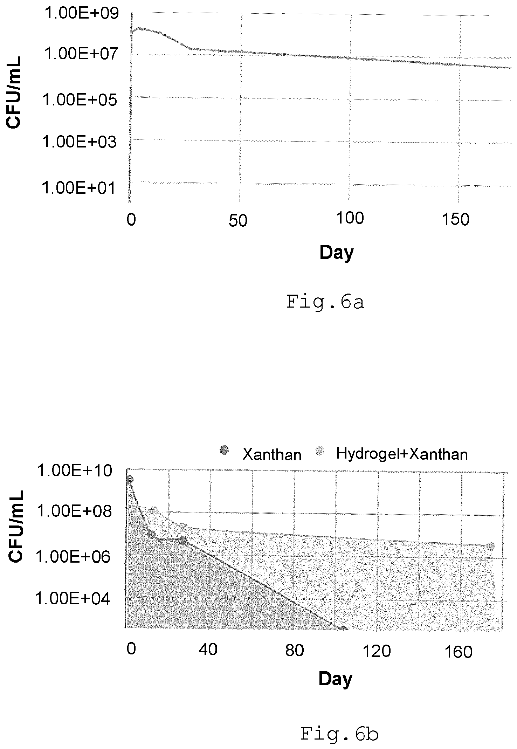

To test the ability of the enzymatically produced hydrogel to preserve the viability of cells, two different bacterial strains (*L. rhamnosus* and *L. lactis*) were encapsulated. The number of viable bacteria was assessed after defined time points. For measuring the number of viable bacteria, 100 um of gel was diluted in PBS and plated on agar plates at respective time points. The results show a slow decrease of viable cells in the gel over the shelf life. Within 174 days only around 2 log was lost (Fig. 6a). In comparison to known hydrogels (containing xanthan as the sole polysaccharide) the stability of the hydrogel of the present invention (cellulose-xanthan hydrogel) was greatly increased (Fig. 6b).

Other parameters measured were the optical stability. The gel presented as a white opaque mass for at least 6 months at room temperature. The mechanical stability was also favorable when xanthan was added. This was tested by turning the tube. The gel did not run off for at least 6 months. The odor was harsh in the first days for *Lb. rhamnosus*, but after several weeks a yogurt-like acidic smell enfolded. *L. lactis* is more neutral in smell. The pH was also measured by using pH strips. It could be shown that the pH decreased slowly (within 4-6 months) from 7,5 to 4,5.

### Example 5: Strain of L. lactis 10123 inhibiting S. aureus

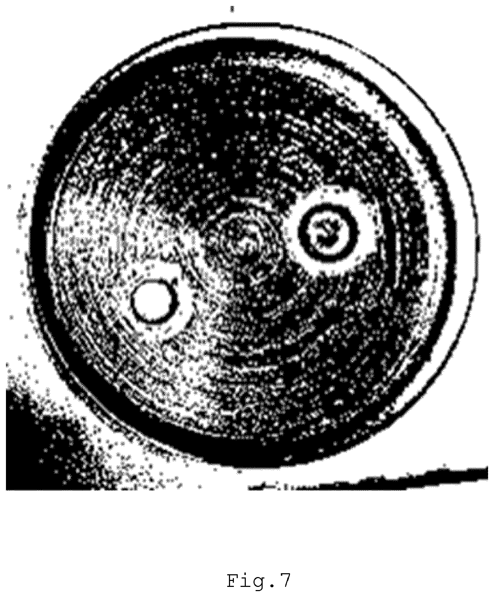

A strain of *L. lactis* was isolated from raw cow's milk in Graz, Styria. A Müller-Hinton agar plate was inoculated with 100 uL of an overnight culture (ONC) of 10^5 *L.lactis.* The plate was inoculated with a swab soaked in PBS with 10^8 CFU/ml *S. aureus* and the ONC was The ONC was poured in a central punching agar plate. After 24h of incubation at 37 degrees inhibition zones were visible for *S. aureus* (Fig. 7) as well as for MRSA, *S. hominis, E. hirae*). No inhibition zones were visible when the cell-free supernatant of a *L*. *lactis* ONC was used.

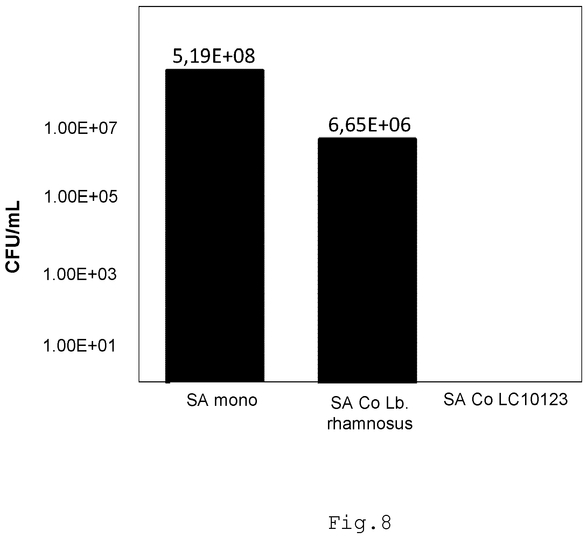

In another set of experiments, the ability to inhibit *S*. *aureus* was also tested in an ONC with 1:1 MRS and LB-Medium at 37 degrees for 24 hours. 10 ml medium was inoculated with 10^6 CFU *S. aureus* and 10^6 CFU *L. lactis* 10123. The culture density was adjusted in comparison to the standard of 0.5 MFU (McFarland Units). *Lb. rhamnosus* was used as a co-culture-agent with *S. aureus* in an ONC. The CFU at the start of the experiment has been verified by counting colonies on the plates, wherein at least 10^5 CFU/ml were detected. The results in Fig. 8 show that after 24 hours, no *S. aureus* was found in the co-culture, whereas in the *S. aureus*-monoculture exhibited over 10^8 CFU/ml *S*. *aureus.* As also shown in literature other tested strains lead a reduction of less than 2 log was achieved (in comparison to a reduction to more than 8 log with *L. lactis* 10123).

### Example 6: Functionality of the 10123 strain after encapsulation

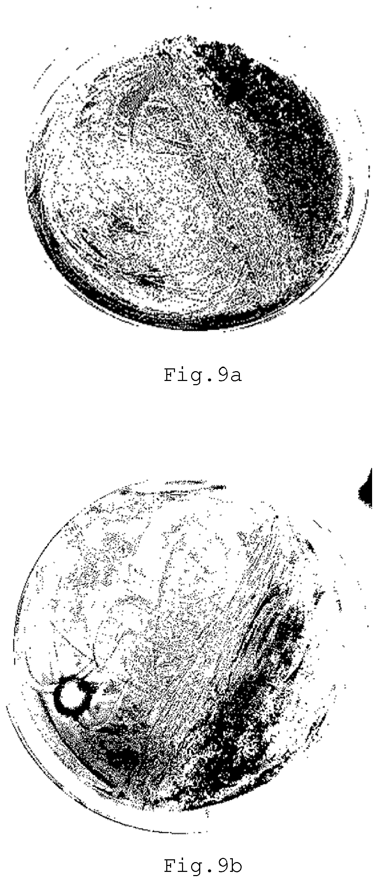

To investigate the preservation of functionality of the L.lactis-strain 10123 after 1 month of storage after encapsulation in hydrogel comprising xanthan at room temperature, a blood agar plate was inoculated with 10^6 CFU *S. aureus* and on one side of the plate 100 um of the gel with 10^7 CFU/ml *L. lactis* was applied on top. The gel itself was applied on *S. aureus* too. The results show that *S. aureus* colonization was reduced on the right side where the gel had been applied (Fig. 9a) and where the gel drop had been applied directly, there was also an inhibition zone visible (Fig 9b).

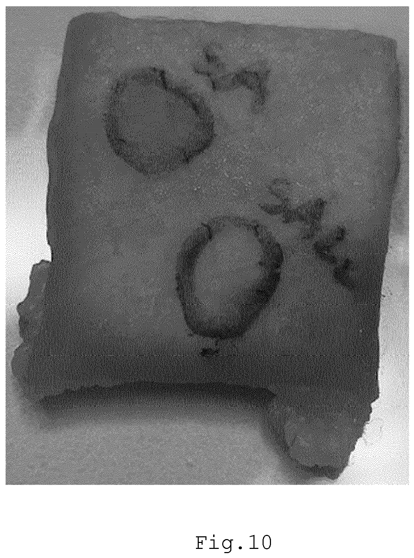

The functionality of the 10123-strain was also shown on a porcine skin model (dermis). A marked spot (left) was inoculated with *S. aureus* and a second marked spot (right) was inoculated with *S. aureus* and *L. lactis.* After 24h of incubation at 37°C a visible difference between the two spots was observable (Fig. 10).

## Claims

1. Method for producing a stable hydrogel-cell composition comprising the steps of:
a) providing a reaction mixture comprising at least one polymerizable substrate and at least one enzyme capable of polymerizing said at least one substrate, and cells, and
b) incubating the mixture of step a) to form a stable hydrogel-cell composition.

2. Method for producing a stable hydrogel composition comprising the steps of:
a) providing a reaction mixture comprising at least one polymerizable substrate and at least one enzyme capable of polymerizing said at least one substrate, and xanthan, and
b) incubating the mixture of step a) to form a stable hydrogel composition.

3. Method according to claim 2, wherein the reaction mixture further comprises cells.

4. Method according to any one of claims 1 to 3, wherein the at least one polymerizable substrate is a sugar phosphate, preferably a hexose phosphate, more preferably a glucose phosphate, even more preferably alpha-D-glucose-1-phosphate.

5. Method according to claim 4, wherein the alpha-D-glucose-1-phosphate is formed by incubating sucrose with sucrose phosphorylase.

6. Method according to any one of claims 1 to 5, wherein the reaction mixture comprises at least one further monosaccharide, oligosaccharide and/or polysaccharide.

7. Method according to claim 6, wherein the at least one further monosaccharide is glucose and/or the at least one further oligosaccharide is a disaccharide, preferably cellobiose.

8. Method according to any one of claims 1 to 7, wherein said at least one enzyme is a phosphorylase, more preferably an oligosaccharide phosphorylase or a polysaccharide phosphorylase.

9. Method according to any one of claims 1 to 8, wherein said at least one enzyme is a cellodextrin phosphorylase.

10. Method according to any one of claims 1 to 9, wherein the reaction mixture comprises at least one further carbohydrate polymer, preferably in an amount of 0.1 to 1 wt% of the reaction mixture, wherein the at least one further carbohydrate polymer is preferably xanthan.

11. Method according to any one of claims 1 to 10, wherein the cells are bacterial cells, fungal cells, animal cells or human cells.

12. Method according to claim 11, wherein the bacterial cells are of the order of Lactobacillales, preferably of the family of Lactobacillaceae and/or of the family of Streptococcaceae, more preferably of the genus Lactococcus and/or of the genus Lactobacillus, wherein the bacterial cells are preferably selected from the group consisting of *Lactococcus lactis*, preferably *Lactococcus lactis* 10123 (DSM 34156), and *Lactobacillus rhamnosus.*

13. Hydrogel-cell composition obtainable by a method according to any one of claims 1 to 12.

14. *Lactococcus lactis* strain 10123 deposited on February 04, 2022, with the DSMZ under deposit number DSM 34156.

15. A kit for producing a stable hydrogel-cell composition comprising at least one polymerizable substrate and at least one enzyme capable of polymerizing said at least one substrate, wherein the at least one polymerizable substrate is a substrate as preferably defined in any one of claims 4 to 7, wherein the at least one enzyme is an enzyme as preferably defined in claims 8 or 9.
